# EUROPEAN PATENT SPECIFICATION

(11) **EP 1 506 007 B1**
(45) Date of publication and mention of the grant of the patent: **22.06.2011**
(21) Application number: 03732933.1
(22) Date of filing: 14.05.2003
(51) Int. Cl.: A61K 39/09

(54) **MUCOSAL COMBINATION VACCINES FOR BACTERIAL MENINGITIS**
KOMBINATIONSIMPFSTOFF GEGEN BAKTERIELLE MENINGITIS, DER ÜBER DIE SCHLEIMHAUT APPLIZIERT WIRD
VACCINS COMBINES DESTINES A ETRE ADMINISTRES AUX MUQUEUSES POUR L'IMMUNISATION CONTRE LA MENINGITE BACTERIENNE

(30) Priority: 14.05.2002 US 380675 P
(43) Date of publication of application: 16.02.2005
(73) Proprietor: Novartis Vaccines and Diagnostics S.r.l., 53100 Siena (SI) (IT)
(72) Inventor: O'HAGAN, Derek, Thomas, Emeryville, CA 94608-2916 (US)
(74) Representative: Marshall, Cameron John
(86) International application number: PCT/IB2003/002648
(87) International publication number: WO 2003/094960

(56) References cited:
- WO-A-02/00249
- WO-A-98/58668
- WO-A-99/42130
- PORRO M ET AL: "A MOLECULAR MODEL OF ARTIFICIAL GLYCOPROTEIN WITH PREDETERMINED MULTIPLE IMMUNODETERMINANTS FOR GRAM-POSITIVE AND GRAM-NEGATIVE ENCAPSULATED BACTERIA" MOLECULAR IMMUNOLOGY, vol. 23, no. 4, 1986, pages 385-392, XP009022921 ISSN: 0161-5890
- UGOZZOLI MILDRED ET AL: "Potency of a genetically detoxified mucosal adjuvant derived from the heat-labile enterotoxin of Escherichia coli (LTK63) is not adversely affected by the presence of preexisting immunity to the adjuvant" JOURNAL OF INFECTIOUS DISEASES, vol. 183, no. 2, 15 January 2001 (2001-01-15), pages 351-354, XP002264403 ISSN: 0022-1899
- GIULIANI MARZIA MONICA ET AL: "Mucosal adjuvanticity and immunogenicity of LTR72, a novel mutant of Escherichia coli heat-labile enterotoxin with partial knockout of ADP-ribosyltransferase activity" JOURNAL OF EXPERIMENTAL MEDICINE, vol. 187, no. 7, 6 April 1998 (1998-04-06), pages 1123-1132, XP002264512 ISSN: 0022-1007
- UGOZZOLI MILDRED ET AL: "Combinations of protein polysaccharide conjugate vaccines for intranasal immunization" JOURNAL OF INFECTIOUS DISEASES, vol. 186, no. 9, 1 November 2002 (2002-11-01), pages 1358-1361, XP002264405 ISSN: 0022-1899

## Description

### FIELD OF THE INVENTION

This application relates to mucosal meningitis vaccines, especially intranasal vaccines.

### BACKGROUND TO THE INVENTION

Meningitis is the inflammation of the tissues which cover the brain and spinal cord. It may have a bacterial cause or a viral cause, with bacterial meningitis generally being more serious.

The main pathogen responsible for bacterial meningitis is *Neisseria meningitidis* (meningococcus), but other relevant pathogens include *Straptococcus pneumoniae* (pneumococcus), *Haemophilus influenzae* (Hib), and *Streptococcus agalactiae* (GBS). *N.mentrigitidis* also causes meningococcal septicaemia, which is the main life-threatening aspect of infection.

Vaccines to protect against Hib infection have been available for many years. A vaccine that protects against serogroup C meningococcus ('MenC') was introduced in several European countries in 1999-2000. A pneumococcal vaccine entered into routine use in America in 2000.

The vaccines against these three pathogens are based on antigenic capsular polysaccharides, with conjugation to carrier proteins being used to enhance the polysaccharides' immunogenicity. These vaccines are administered by injection, although investigations into mucosal delivery have boon described for mice *e.g*, reference 1 describes the intranasal administration of Hib conjugate vaccines and reference 2 describes intranasal administration of MenC conjugate vaccines. (see also ref. 3). Mucosal delivery of vaccines represents an attractive approach to overcome the problem of the high number of injections administered to young children. In addition, as most pathogens initially infect at mucosal surfaces, inducing mucosal immunity at the site of infection would likely contribute to optimal protective immunity.

Intranasal and oropharyngeal delivery of vesicle-based vaccines against serogroups B meningococcus ('MenB') has also been described [*e.g*. ref 4], as has the intranasal delivery of *B.pertussis* bacteria which express *N.meningitidis* transferrin-binding protein B [5]. Reference 6 describes the intranasal delivery of pneumococcal conjugates vaccines [see also refs. 7 & 8].

It is an object of the invention to provide improvements in the mucosal delivery of meningitis vaccines.

### DISCLOSURE OF THE INVENTION

The invention provides a compositions for mucosal delivery as defined in appended claim 1. The invention also provides the composition of appended claim 18.

Combining different antigens reduces the number of different doses which need to be administered in order to immunise against multiple pathogens. This is typically seen as an advantage for injectable vaccines, where the number of painful injections is reduced, but it is less important in mucosal vaccines (*e.g*. intranasal vaccines) because of the lower discomfort levels associated with delivery. However, combined antigen compositions are advantageous even for mucosal delivery because patient compliance is improved and transport/storage of medicines is facilitated.

Although combining antigens into a single dose is attractive [*e.g*. refs. 9 to 12], it presents difficulties due to interactions between the various components once combined, particularly in liquid formulations [13]. Issues which arise include antigen interference, antigen competition [14,15], antigen degradation, epitope suppression, and adjuvant compatibility. Quality control of mixtures is also more difficult. Furthermore, existing knowledge on combining antigens focuses on injectable, not mucosal, vaccines.

Despite these difficulties, the inventors have surprisingly found that antigens from *Haemophilus influenzae, Neisseria meningitidis* and/or *Streptococcus pneumoniae* can be combined for mucosal delivery without the negative consequences which would haven been expected. Combining antigens from these three organisms is also advantageous because it allows a single dose to deal with three separate causes of a common disease, namely bacterial meningitis. Combined meningitis vaccines of this type have previously been reported [16], but mucosal administration was not reported.

### Mucosal delivery

The composition of the invention is for mucosal delivery.

Of the various mucosal delivery options available, the intranasal route is the most practical as it offers easy access with relatively simple devices that have already been mass produced. In addition, intranasal immunisation appears to be more potent that alternative routes. Thus the preferred route for mucosal delivery is the intranasal route, and the composition of the invention is preferably adapted for intranasal administration, such as by nasal spray, nasal drops, gel or powder [*e.g*. refs 17 & 18].

Alternative routes for mucosal delivery of the vaccine are oral, intragastric, pulmonary, intestinal, rectal, ocular, and vaginal routes.

### (a) Haemophilus Influenzae antigen

The *H.influenzae* antigen in the composition is a capsular saccharide antigen. Saccharide antigens from *H.influenzae* b are well known.

Advantageously, the Hib saccharide is covalently conjugated to a carrier protein, for example in order to enhance its immunogenicity, especially in children. The preparation of polysaccharide conjugates in general, and of the Hib capsular polysaccharide in particular, is well documented [*e.g*. references 19 to 27 *etc*.]. The invention may use any suitable Hib conjugate.

The saccharides moiety of the conjugate may be a polysaccharide (*e.g*. full-length polyribosylribitol phosphate PRP)), but it is preferred to hydrolyse polysaccharides (*e.g*. by acid hydrolysis) to form oligosaccharides (*e.g*. MW from ∼1 to ∼5 kDa). If hydrolysis is performed, the hydrolysate may be sorted by size in order to remove oligosaccharides which are too short to be usefully immunogenic. Size-separated oligosaccharides are preferred saccharide antigens.

Preferred carrier proteins are bacterial toxins or toxoids, such as diphtheria or tetanus toxoids. These are commonly used in conjugate vaccines. The CRM197 diphtheria toxoid is particularly preferred [28]. Other suitable carrier proteins include the *N.meningitidis* outer membrane protein [29], synthetic peptides [30,31], heat shock proteins [32,33], pertussis proteins [34,35], protein D from *H.influenzae* [36], cytokines [37], lymphokines [37], hormones [37], growth factors [37], toxin A or B from *C.difficile* [38], iron-uptake proteins [39] *etc.* It is possible to use mixtures of carrier proteins.

The saccharide moiety may be conjugated to the carrier protein directly or via a linker. Direct linkage may be achieved by oxidation of the polysaccharide followed by reductive amination with the protein, as described in, for example, refs. 40 & 41. Linkage via a linker group may be made using any known procedure, for example, the procedures described in refs. 42 & 43. Suitable linkers include carbonyl, adipic acid, B-propionamido [44], nitrophenyl-ethylamine [45], haloacyl halides [46], glycosidic linkages [47], 6-aminocaproic acid [48], ADH [49], C₄ to C₁₂ moieties [50] *etc.*

The saccharide will typically be activated or functionalised prior to conjugation. Activation may involve, for example, cyanylating reagents such as CDAP (*e.g*. 1-cyano-4-dimethylamino pyridinium tetrafluoroborate [51, 52]. Other suitable techniques use carbodiimides, hydrazides, active esters, norborane, p-nitrobenzoic acid, N-hydroxysuccinimide, S-NHS, EDC, TSTU; see also the introduction to reference 53). Reductive amination is a preferred technique.

A preferred conjugate comprises the Hib saccharide covalently linked to CRM197 via adipic acid succinic diester [54, 55].

Compositions of the invention may comprise more than one Hib antigen.

### (b) Neisseria meningitidis antigen

The *N.meningitidis* antigen in the composition is a capsular saccharide antigen (*e.g*. from serogroups A, C, W135 or Y). Saccharide antigens from *N.meningitidis* are well known. Where the antigen is from serogroup B, however, it is preferred that the antigen is a protein antigen. This is because the native capsular polysaccharide of MenB contains self-antigens. If a saccharide antigen is to be used from serogroup B, it is preferred to use a modified saccharide antigen [*e.g*. refs. 56, 57, 58] *e.g*. one modified by N-propionylation. Chemical modification of saccharides from other serogroups is also possible.

The saccharide is preferably an oligosaccharide i.e. a fragment of a capsular polysaccharide, Polysaccharides may be manipulated to give shorter oligosaccharides and these may be obtained by purification and/or sizing of the native polysaccharide (*e.g* by hydrolysis in mild acid, by heating, by sizing chromatography *etc*.). Preferred MenC oligosaccharides are disclosed in references 59 & 60.

The saccharide is conjugated to a carrier protein as described above.

Compositions of the invention may comprise more than one meningococcal antigen. It may be preferred to include capsular saccharide antigens from at least two (i.e. 2, 3 or 4) of serogroups A, C, W135 and Y *of N.meningitidis* [61].

Where a mixture comprises capsular saccharides from both serogroups A and C, it is preferred that the ratio (w/w) of MenA saccharide:MenC saccharide is greater than 1 (*e.g.* 2:1, 3;1 , 4;1 , 5:1, 10:1 or higher). Surprisingly, improved immunogenicity of the MenA component has been observed when it is present in excess (mass/dose) to the MenC component [61].

Where a mixture comprises capsular saccharides from serogroup W135 and at least one of serogroups A, C and Y, it has surprisingly been found that the immunogenicity of the McnW135 saccharide is greater when administered in combination with the saccharide(s) from the other serogroup(s) than when administered alone (at the same dosage *etc*.) [61]. Thus the capacity of the MenW135 antigen to elicit an immune response is greater than the immune responses elicited by an equivalent amount of the same antigen when delivered without association with the antigens from the other serogroups. Such enhanced immunogenicity can be determined by administering the MenW135 antigen to control animals and the mixture to test animals and comparing antibody titres against the two using standard assays such as bactericidal titres, radioimmunoassay and ELISAs *etc.* Vaccines comprising synergistic combinations of saccharides from serogroup W135 and other serogroups are immunologically advantageous as they allow enhanced anti-W135 responses and/or lower W135 doses.

Where a protein antigen from serogroup B is used, it is preferred to use one of the proteins disclosed in references 62 to 71]. Preferred protein antigens comprise the '287' protein or derivatives (*e.g*. ΔG287).

It is also possible to use an outer membrane vesicle (OMV) antigen for serogroup B *[e.g.* 72, 73].

Compositions of the invention may comprise more than one meningococcal antigen.

### (c)Streptococcus pneumoniae antigen

The *S.pneumoniae* antigen in the composition is a capsular saccharide antigen which is conjugated to a carrier protein as described above [*e.g*. 74, 75,76].

It is preferred to include saccharides from more than one serotype of *S.pneumoniae.* For example, mixtures of polysaccharides from 23 different serotype are widely used, as are conjugate vaccines with polysaccharides from between 5 and 11 different serotypes [77]. For example, PrevNar™ contains antigens from seven serotypes (4, 6B, 9V, 14, 18C, 19F, and 23F) with each saccharide individually conjugated to CRM197 by reductive amination.

Compositions of the invention may thus comprise more than one pneumococcal antigen.

### Further components - adjuvants

Compositions of the invention comprise a mucosal adjuvant. Mucosal adjuvants include, but are not limited to, (A) *E.coli* heat-labile enterotoxin ("LT"), or detoxified mutants thereof, such as the K63 or R72 mutants [*e.g*. Chapter 5 of ref. 78]; (B) cholera toxin ("CT'), or detoxified mutants thereof [*e.g*. Chapter 5 of ref. 78]; or (C) microparticles (*i.e*. a patticle of ∼100nm to ∼150µm in diameter, more preferably ∼200nm to ∼30µm in diameter, and most preferably ∼500nm to ∼10µm in diameter) formed from materials that are biodegradable and non-toxic (*e.g*. a polyp-hydroxy acid), a polyhydroxybutyric acid, a polyorthoester, a polyanhydride, a polycaprolactone *etc.);* (D) a polyoxyethylene ether or a polyoxyethylene ester [79]; (E) a polyoxyethylene sorbitan ester surfactant in combination with an octoxynol [80] or a polyoxyethylene alkyl ether or ester surfactant in combination with at least one additional non-ionic surfactant such as an octoxynol [81]; (F) chitosan [*e.g*. 82]; (G) an immunostimulatory oligonucleotide (*e.g*. a CpG oligonucleotide), (H) double stranded RNA; (I) a saponin [83]; (J) monophosphoryl lipid A mimics, such as aminoalkyl glucasaminide phosphate derivatives *e.g*. RC-529 [84]; or (K) polyphosphazene (PCPP). Other mucosal adjuvants are also available [*e.g*. see chapter 7 of ref, 85].

The compositions of the invention contain bacterial ADP-ribosylating toxins or their mutants as mucosal adjuvants. For example, cholera toxin (CT) or *E.col**i*** heat labile toxin (LT) are potent mucosal adjuvants, as are their detoxified counterparts [86]. CT and LT are homologous and are typically interchangeable. Detoxification of the CT or LT may be by chemical or, preferably, by genetic means. Suitable examples include LT having a lysine residue at amino acid 63 ['LT-K63' - ref. 87], and LT having an arginine residue at amino acid 72 ['LT-R72' - ref. 88]. Other suitable mutants include LT with a tyrosine at residue 63 ['Y63' - ref. 89] and the various mutants disclosed in reference 90, namely D53, K97, K104 and S106, as well as combinations thereof (*e.g*. LT with both a D53 and a K63 mutation).

The composition may comprise a bioadhesive [91,92] such as esterified hyaluronic acid microspheres [93] or, in preferred embodiments, a mucoadhesive selected from the group consisting of cross-linked derivatives of poly(acrylic acid), polyvinyl alcohol, polyvinyl pyrollidone, polysaccharides and carboxymethylcellulose.

Compositions of the invention may comprise more than one mucosal adjuvant.

### Further components - antigens

The combination of antigens from *H.influenzae, N.meningitidis* and *S.pneumoniae* is advantageous because they all cause bacterial meningitis. Antigens which induce immune responses against further organisms may also be included in compositions of the invention *e.g*.
- antigens from *Helicobacter pylori* such as CagA [94 to 97], VacA [98, 99], NAP [100, 101, 102], HopX *[e.g.* 103], HopY *[e.g.* 103] and/or urease.
- an antigen from hepatitis A virus, such as inactivated virus [e.g. 104, 105].
- an antigen from hepatitis B virus, such as the surface and/or core antigens [*e.g*. 105, 106].
- an antigen from hepatitis C virus [*e.g*. 107].
- an antigen from *Bordetella pertussis,* such as pertussis holotoxin (PT) and filamentous haemagglutinin (FHA) from *B. pertussis,* optionally also in combination with pertactin and/or agglutinogens 2 and 3 (*e.g*., refs. 108 and 109].
- a diptheria antigen, such as diphtheria toxoid [*e.g*., chapter 3 of ref. 117] *e.g.* the CRM₁₉₇ mutant [*e.g*. 83].
- a tetanus antigen, such as a tetanus toxoid [*e.g*., chapter 4 of ref. 114].
- an antigen from *N.gonorrhoeae* [*e.g.* 62 to 65].
- an antigen from *Chlamydia pneumoniae* [*e.g.* 110, 111, 112, 113, 114, 115, 116].
- an antigen from *Chlamydia trachomatis* [*e.g. 117].*
- an antigen from *Porphyromonas gingivalis [e.g.* 118].
- polio antigen(s) [*e.g*. 119, 120] such as IPV or OPV.
- rabies antigen(s) *[e.g.* 121] such as lyophilised inactivated virus [*e.g*.122, RabAvert™].
- measles, mumps and/or rubella antigens [e.g. chapters 9, 10 & 11 of ref. 123].
- influenza antigen(s) [*e.g*. chapter 19 of ref. 123], such as the haemagglutinin and/or neuraminidase surface proteins.
- antigen(s) from a paramyxovirus such as respiratory syncytial virus (RSV [124,125]) and/or parainfluenza virus (PIV3 [126]).
- an antigen from *Moraxella catarrhalis* [*e.g.* 127].
- an antigen from *Streptococcus agalactiae* (group B streptococcus) [*e.g*. 128, 129].
- an antigen from *Streptococcus pyogenes* (group A streptococcus) [e.g. 129, 130, 131].
- an antigen from *Staphylococcus aureus [e.g.* 132].

The composition may comprise one or more of these further antigens.

Where a conjugate is present, the composition may also comprise free carrier protein [133].

It is preferred that the composition does not include whole bacteria (whether intact or lysed).

Compositions of the invention may comprise proteins which mimic saccharide antigens *e.g*. mimotopes [134] or anti-idiotype antibodies. These may replace individual saccharine components, or may supplement them. As an example, the vaccine may comprise a peptide mimic of the MenC [135] or the MenA [136] capsular polysaccharide in place of the saccharide itself.

Compositions of the invention may comprise nucleic acid for 'genetic immunisation' [*e.g*. 137]. The nucleic acid will encode a protein component of the composition and may replace individual protein components (including those of the previous paragraph), or may supplement them. As an example, the vaccine may comprise DNA that encodes a tetanus toxin.

### Further components - formulation

The composition of the invention preferably includes a pharmaceutically acceptable carrier.

'Pharmaceutically acceptable carriers' include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, trehalose [138] lipid aggregates (such as oil droplets or liposomes), and inactive virus particles. Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. The carrier will be compatible with mucosal administration. A thorough discussion of pharmaceutically acceptable excipients is available in *Remington's Pharmaceutical Sciences.*

The composition of the invention is preferably sterile.

The composition of the invention is preferably buffered.

The composition of the invention is preferably pyrogen-free.

The composition of the invention may be packaged with its components (a), (b) and/or (c) in admixture, or these components may remain separate until they are to be administered to a patient, at which stage they will be combined. Where separate, the individual components may each be in lyophilised form or in solution/suspension. Where mixed, the components will all be in lyophilised form or all in solution/suspension. Lyophilised components will be re-suspended (*e.g*. in buffer) prior to administration to a patient. Components such as adjuvants may be present in the buffer or in the lyophilised material.

### Immunogenic compositions

The composition of the invention is preferably an immunogenic composition (*e.g*. a vaccine). Formulation of vaccines based on saccharides or saccharide-protein conjugates is well known in the art.

Immunogenic compositions comprise immunologically effective amounts of antigens, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

### Methods of treatment

Once formulated, the compositions of the invention can be administered directly to a patient, which will generally be a human. The human is preferably a child or a teenager. A further preferred class of patient is an adult woman, and particularly a woman of child-bearing age or a pregnant woman. Compositions of the invention are particularly suited for passively immunising children via the maternal route.

Antigens in the composition induce immune responses against certain bacteria. These immune responses are preferably protective i.e. they protect the patient from later infection by the bacteria. Thus the compositions of the invention are preferably used for prophylaxis (i.e. to prevent infection), although they may also be used for therapeutic purposes (i.e. to treat disease after infection). The immune responses preferably involve the production of bactericidal antibodies in the patient.

The invention provides a vaccine of the invention for use in a method of raising an immune response in a patient, comprising administering to a patient the vaccine via a mucosal route (*e.g*, intranasally). The immune response is preferably protective against bacterial meningitis and/or bacteremia caused by *Haemophilus influenzae, Neisseria meningitidis* and/or *Streptococcus pneumoniae.* The individual antigenic components of the compositions are preferably administered simultaneously and in combination. In other embodiments, however, they may be administered separately, either simultaneously or sequentially. When they are administered separately, the components are preferably delivered to the same mucosal surface.

The invention also provides a composition of the invention for use as a medicament.

The invention also provides the use of a composition of the invention in the manufacture of a medicament for immunising a patient.

These methods and uses may involve a prime/boost regime. The methods and uses may involve a priming dose which will be followed by a booster dose, where the booster dose may be by a mucosal or parenteral route. Similarly, the methods and uses may raise a booster response in a patient that has already been immunologically primed, where the primer dose may have been by a mucosal or parenteral route. Booster doses may comprise fewer antigens than priming doses *e.g*. they may use a single antigen.

Dosage treatment at priming and/or boosting may be a single dose or a multiple dose schedule. Compositions of the invention may be presented in unit dose form.

### Manufacturing methods

The invention provides a method for producing a composition of the invention as defined in appended claim 19.

### BRIEF DESCRIPTION OF THE DRAWINGS

**Figure 1** shows geometric mean serum IgG antibody titres against MenC. The label on the X axis shows the adjuvant which was used. The plain left-hand column in each pair shows data obtained after administration of the saccharide antigen on its own, whereas the shaded right-hand column shows data obtained after administration of combined saccharide antigens. Figure 1A shows anti-MenC responses and Figure 1B shows anti-Hib responses. Error bars are standard deviations within one standard error.
**Figure 2** shows bactericidal antibody titres against MenC. As in Figure 1, shaded data were obtained using combined saccharide antigens.
**Figure 3** shows anti-MenC IgA titres from nasal wash. As before, shaded data were obtained using combined saccharide antigens.

### MODES FOR CARRYING OUT THE INVENTION

### Combined Hib/MenC composition

*Neisseria meningitidis* serogroup C capsular oligosaccharide was produced by selective end-reducing group activation of sized oligosaccharide. The same method was used for *Haemophilus influenzae* type B. The saccharides were conjugated to protein carrier CRM197 through a hydrocarbon spacer [139] (Chiron Siena, Italy). The conjugates were diluted in phosphate-buffered saline (PBS) and combined with (i) mutant E*.coli* heat-labile enterotoxin LTK63 or LTR72), (ii) aluminium hydroxide (Superfos Biosector a/s) or (iii) Cholera toxin (CT) from Sigma. For combined administration, these formulations were mixed prior to use.

### Mucosal administration of the composition

Two identical administration studies were performed simultaneously. Groups of 10 female BALB/C mice 6-10 weeks old were immunised intranasally with 10µg of MenC or Hib alone, combined with CT (1 µg), or with the LT mutants (1µg and 10µg). For comparison, an additional group of mice was immunised IM with 10 µg of MenC or Hib adsorbed to Alum. The compositions were prepared on the same day as immunisation and mice were immunised on Days 0, 21, and 35. 50µl of the compositions were injected into the thigh or instilled into alternate nostrils in unaneesthetised mice. Blood samples were taken on Day 49 along with terminal nasal wash samples (NW).

To evaluate if the immunogenicity of the conjugates was impaired when the two were mixed, a third study was performed concurrently in which the two vaccines were administered simultaneously to the same groups of mice, at the same doses and regimen described above.

### Immunological responses to the compositions

Antibody responses against the MenC conjugate were measured by ELISA using a modified procedure as previously described [140]. Briefly, ELISA plates were coated with adipidic dihydrazide-derivatised MenC saccharide over night at 4°C. Specific antibodies were developed with goat anti Mouse IgG-horseradish peroxidase conjugate. MenC IgG antibody titres for the test samples and the internal control were expressed as the reciprocal of the serum dilution giving OD=1.0. Each serum sample was assayed in duplicate, and the average value was used to calculate the geometrical mean and the standard deviation within one standard error. The antibody responses against Hib PRP were determined similarly to the MenC ELISA, except that the plates were coated with BSA conjugated PRP (PRP-BSA). Titres were expressed as OD₄₅₀ₙₘ for serum diluted 1:50.

Nasal washes were assayed for IgA anti-MenC using a bioluminescent assay (BIA) [141]. Briefly, identical reagents and coating procedure to measure serum IgG against MenC was used. Then, a biotinylated Goat anti-Mouse IgA specific was added as a first antibody. Titres represent the logarithmic dilution values extrapolated from the log RLU data at the cutoff value calculated at least two standard deviations above mean background.

Complement-mediated bactericidal activity against MenC bacteria was measured in pooled serum samples as previously described [140]. Titres were determined by calculating the serum dilution showing a 50% reduction in the number of CFU after I hour incubation.

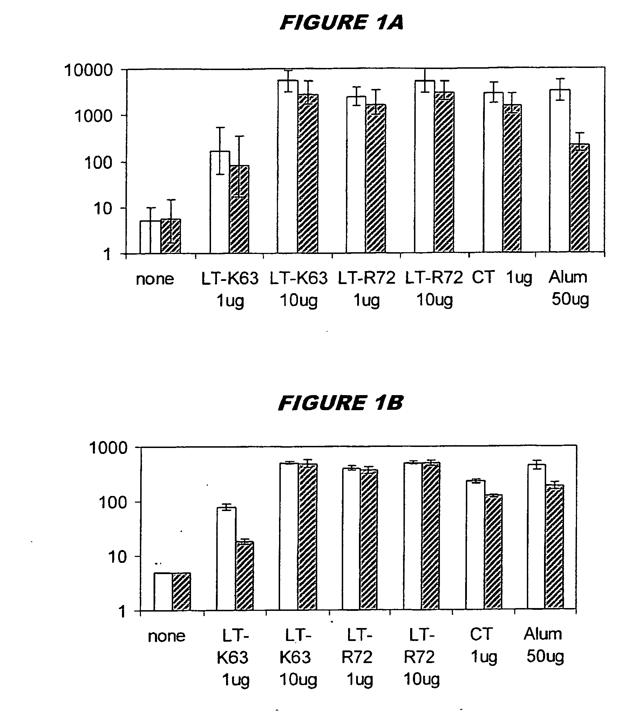

Figure 1A shows geometric mean serum IgG antibody titres against MenC, either alone (plain columns) or in combination with Hib antigen (shaded columns). The serum antibody responses elicited by both LT mutants were significantly higher than those obtained with the antigen alone. LTR72 exhibited a higher adjuvanticity than LTK63 at lower doses. Most notably, the antibody responses induced by intranasal immunisation with both LT mutants were comparable to those achieved with wild-type CT, or those induced by intramuscular immunisation with alum-adjuvanted vaccine. Importantly, the addition of a second conjugated saccharide antigen did not adversely affect the antibody responses to either antigen.
Figure 1B shows geometric mean serum IgG antibody titres against Hib PRP saccharide. As for for MenC, antibody responses induced by either LT mutant were higher than those achieved with the antigen alone. Again, LTR72 showed a better adjuvanticity. Comparable titres were induced in mice immunised intranasally with LT mutants and by alum-adjuvanted vaccine by intramuscular immunisation. In addition, there was no evidence of competition following combined intranasal immunisation with the two saccharide conjugate vaccines, with responses induced against Hib when in combination with MenC being comparable to the responses induced by immunisation with Hib alone.

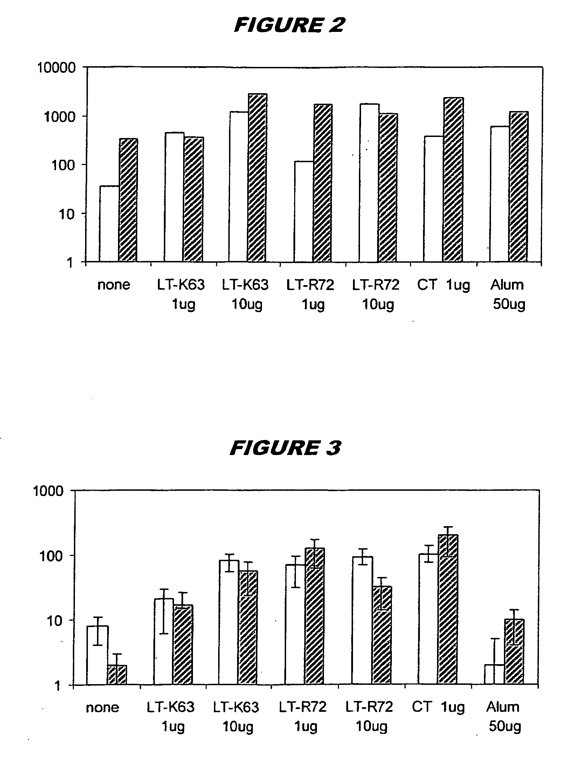

The levels of bactericidal antibodies induced by intranasal immunisation with LT mutants closely correlate with the ELISA serum IgG responses, and were again comparable to the responses induced by CT, or intramuscular immunisation with alum adsorbed vaccine (Figure 2)

Samples obtained from nasal wash following intranasal immunisation with MenC with either LT mutant showed higher IgA titers than those obtained by intranasal immunisation in the absence of adjuvants (Figure 3). As expected, intramuscular immunisation elicited very low IgA titers.

### Conclusion

Potent serum antibody responses against *N.meningitidis* and *H.influenzae* can be induced by intranasal immunisation with conjugate vaccines in combination with mucosal adjuvants. Moreover, for the MenC antigen, the antibodies induced by intranasal immunisation had potent bactericidal activity, which is known to correlate with protective immunity [142]. In addition, IgA responses in the nasal cavity were induced only in animals immunised through the intranasal route. Inducing secretory immunity is important because the upper respiratory tract is the portal of entry for several pathogens, including *N.meningitidis* and *H.influenzae.*

Based on antibody titres obtained with conjugate vaccines given alone and in combination, and on the bactericidal activity measured against MenC, the combination of two vaccines co-administered with mucosal adjuvant did not negatively influence the antibody responses against MenC or Hib. The results thus suggest that intranasal immunisation is an effective route of immunisation for polysaccharide-protein conjugate vaccines in combination with mucosal adjuvants such as LT mutants.

The same dose of LT mutants was sufficient to significantly enhance the immunogenicity of both conjugate vaccines administered simultaneously. This is particularly important as it would reduce the amount of adjuvant needed and the risks associated with potential toxicity. Importantly, pre-existing immunity against the LTK63 mutant does not affect the ability of the mutant to act as an adjuvant for a second antigen [2]. Furthermore, the potency of mucosally-delivered vaccines may be further improved by formulating the vaccines in bioadhesive delivery systems [91].

In conclusion, combining polysaccharide-protein conjugate vaccines with LT mutants for intranasal immunisation is an effective approach to mucosal immunisation for pediatric use.

It will be understood that the invention is described above by way of example only and modifications may be made whilst remaining within the scope of the invention, which is defined in the appended claims.

### REFERENCES

[1] Bergquist et al. (1998) APMIS 106:800-806.
[2] Ugozzoli et al. (2001) J. Infect. Dis. 183:351-354.
[3] Wenzel et al. (1973) J. Infect Dis, 128:31-40.
[4] Katial et al. (2002) Infect. Immun. 70:702-707.
[5] Coppens et al. (2001) Infect. Immun. 69:5440-5446.
[6] International patent application WO00/53221.
[7] Yamamoto et al. (1998) J Immunol. 161:4115-4121.
[8] Wu et al. (1997) J. Infect. Dis. 175:839-
[9] Corbel (1994) Biologicals 22:353-360.
[10] Rappuoli et al. European Commission COST/STD Initiative, Report of Expert Panel VIII
[11] Pichichero (2000) Pediatr Clin North Am 47:407-426.
[12] International patent application WO02/00249.
[13] Corbel (1994) Dev. Biol. Stand. 87:113-124.
[14] Eskola et al. (1995) "Hib antibody responses after two doses of combined DTPa-Hib conjugate vaccine as compared to separate injections" in Abstract Book of the 35th Interscience Conference on Antimicrobial Agents and Chemotherapy, San Francisco, 17-20 Sep. 1995.
[15] Schmitt et al. (2001) Pediatr Infect Dis J 20:767-774.
[16] US patent 6,251,401.
[17] Almeida & Alpar (1996) J. Drug Targeting 3:455-467.
[18] Agarwal & Mishra (1999) Indian J Exp Biol 37:6-16.
[19] Lindberg (1999) Vaccine 17 Suppl 2:S28-36.
[20] Buttery & Moxon (2000) J R Coll Physicians Lond 34:163-168.
[21] Ahmad & Chapnick (1999) Infect Dis Clin North Am 13:113-33, vii.
[22] Goldblatt (1998) J. Med. Microbiol. 47:563-567.
[23] European patent 0 477 508.
[24] US patent 5,306,492.
[25] WO98/42721.
[26] Dick et al. in Conjugate Vaccines (eds. Cruse et al.) Karger, Basel, 1989, 10:48-114.
[27] Hermanson Bioconjugate Techniques, Academic Press, San Diego CA (1996).
[28] *Research Disclosure,* 453077 (Jan 2002)
[29] EP-A-0372501
[30] EP-A-0378881
[31] EP-A-0427347
[32] WO93/17712
[33] WO94/03208
[34] WO98/58668
[35] EP-A-0471177
[36] WO00/56360
[37] WO91/01146
[38] WO/0061761
[39] WO01/72337
[40] US patent 4,761,283
[41] US patent 4,356,170
[42] US patent 4,882,317
[43] US patent 4,695,624
[44] WO00/10599
[45] Gever et al., Med. Microbiol. Immunol, 165 : 171-288 (1979).
[46] US patent 4,057,685.
[47] US patents 4,673,574; 4,761,283; 4,808,700.
[48] US patent 4,459,286.
[49] US patent 4,965,338
[50] US patent 4,663,160.
[51] Lees et al. (1996) Vaccine 14:190-198.
[52] WO95/08348.
[53] International patent application WO98/42721
[54] Kanra et al. (1999) The Turkish Journal of Paediatrics 42:421-427.
[55] Ravenscroft et al. (2000) Dev Biol (Basel) 103: 35-47.
[56] US patent 4,727,136.
[57] US patent 5,811,102.
[58] Jennings (1988) Adv Exp Med Biol 228:495-550.
[59] Costantino et al. (1992) Vaccine 10:691-698.
[60] Costantino et al. (1999) Vaccine 17:1251-1263/
[61] UK patent application 0115176.0.
[62] WO99/24578
[63] WO99/36544
[64] WO99/57280
[65] WO00/22430
[66] Tettelin et al. (2000) Science 287:1809-1815
[67] Pizza et al. (2000) Science 287:1816-1820
[68] WO00/66741
[69] WO00/71725
[70] WO01/64922
[71] WO01/64920
[72] WO01/52885
[73] Bakke et al. (2001) Infect Immun 69(8):5010-5015.
[74] Watson (2000) Pediatr Infect Dis J 19:331-332.
[75] Rubin (2000) Pediatr Clin North Am 47:269-285, v.
[76] Jedrzejas (2001) Microbiol Mol Biol Rev 65:187-207.
[77] Zielen et al. (2000) Infect. Immun. 68:1435-1440.
[78] Del Giudice et al. (1998) Molecular Aspects of Medicine, vol. 19, number 1.
[79] International patent application WO99/52549.
[80] International patent application WO01/21207.
[81] International patent application WO01/21152.
[82] International patent application WO99/27960.
[83] International patent application WO00/62800.
[84] Johnson et al. (1999) Bioorg Med Chem Lett 9:2273-2278.
[85] Vaccine design: the subunit and adjuvant approach, eds. Powell & Newman, Plenum Press 1995 (ISBN 0-306-44867-X.
[86] Del Guidice et al. (1998) Molecular Aspects of Medicine 19:1-70.
[87] WO93/13202
[88] WO98/18298
[89] Park et al. (2000) Exp. Mol. Med. 32:72-8.
[90] Fontana et al. (1995) Infect. Immun. 63:2356-2360.
[91] International patent application WO00/50078.
[92] International patent application WO99/62546.
[93] Singh et al. (2001) J. Cont. Rele. 70:267-276.
[94] Covacci & Rappuoli (2000) J. Exp. Med. 19:587-592.
[95] WO93/18150.
[96] Covacci et al. (1993) Proc. Natl. Acad. Sci. USA 90: 5791-5795.
[97] Tummuru et al. (1994) Infect. Immun. 61:1799-1809.
[98] Marchetti et al. (1998) Vaccine 16:33-37.
[99] Telford et al. (1994) J. Exp. Med 179:1653-1658.
[100] Evans et al. (1995) Gene 153:123-127.
[101] WO96/01272 & WO96/01273, especially SEQ ID NO:6.
[102] WO97/25429.
[103] WO98/04702.
[104] Bell (2000) Pediatr Infect Dis J 19:1187-1188.
[105] Iwarson (1995) APMIS 103:321-326.
[106] Gerlich et al. (1990) Vaccine 8 Suppl:S63-68 & 79-80.
[107] Hsu et al. (1999) Clin Liver Dis 3:901-915.
[108] Gustafsson et al. (1996) N. Engl. J Med. 334:349-355.
[109] Rappuoli et al. (1991) TIBTECH 9:232-238.
[110] WO02/02606.
[111] Kalman et al. (1999) Nature Genetics 21:385-389.
[112] Read et al. (2000) Nucleic Acids Res 28:1397-406.
[113] Shirai et al. (2000) J. Infect. Dis. 181 (Suppl 3):S524-S527.
[114] International patent application WO99/27105.
[115] International patent application WO00/27994.
[116] International patent application WO00/37494.
[117] International patent application WO99/28475.
[118] Ross et al. (2001) Vaccine 19:4135-4142.
[119] Sutter et al. (2000) Pediatr Clin North Am 47:287-308.
[120] Zimmerman & Spann (1999) Am Fam Physician 59:113-118, 125-126.
[121] Dreesen (1997) Vaccine 15 Suppl:S2-6.
*[122]* MMWR Morb Mortal Wkly Rep 1998 Jan 16;47(1):12, 19.
[123] Vaccines (1988) eds. Plotkin & Mortimer. ISBN 0-7216-1946-0.
[124] Anderson (2000) Vaccine 19 Suppl 1:S59-65.
[125] Kahn (2000) Curr Opin Pediatr 12:257-252.
[126] Crowe (1995) Vaccine 13:415-421.
[127] McMichael (2000) Vaccine 19 Suppl 1:S101-107.
[128] Schuchat (1999) Lancet 353(9146):51-6.
[129] International patent application PCT/GB01/04789.
[130] Dale (1999) Infect Dis Clin North Am 13:227-43, viii.
[131] Ferretti et al. (2001) PNAS USA 98: 4658-4663.
[132] Kuroda et al. (2001) Lancet 357(9264):1225-1240; see also pages 1218-1219.
[133] WO96/40242
[134] Charalambous & Feavers (2001) J Med Microbiol 50:937-939.
[135] Westerink (2001) Int Rev Immunol 20:251-261.
[136] Grothaus et al. (2000) Vaccine 18:1253-1263.
[137] Donnelly et al. (1997) Annu Rev Immunol 15:617-648.
[138] WO00/56365
[139] Constatino et al. (1992) Vaccine 10:691-698.
[140] Dan et al. (1998) Clin. Diagn. Lab. Immunol. 5:479-485.
[141] Ugozzoli et al. (1998) Immunology 93:563-571.
[142] Goldschneider et al. (1969) J Exp Med. 129:1367-1384.

## Claims

1. A composition for mucosal delivery comprising a mucosal adjuvant and two or more of the following: (a) an antigen which induces an immune response against *Haemophilus* influenzae, wherein the antigen is a *Haemophilus influenzae* capsular saccharide conjugated to a carrier protein; (b) an antigen which induces an immune response against *Neisseria meningitidis,* wherein the antigen is a *Neisseria meningitidis* capsular saccharide conjugated to a carrier protein; and (c) an antigen which induces an immune response against *Streptococcus pneumoniae,* wherein the antigen is a *Streptococcus pneumoniae* capsular saccharide conjugated to a carrier protein; and further wherein the mucosal adjuvant is a bacterial ADP-ribosylating toxin or a detoxified mutant thereof.

2. The composition of claim 1, adapted for intranasal administration.

3. The composition of claim 2, in the form of a nasal spray, nasal drops, a gel or a powder.

4. The composition of any preceding claim, wherein the *H. influenzae* capsular saccharide saccharide antigen is an oligosaccharide.

5. The composition of any preceding claim, wherein the *N.meningitidis* antigen is a capsular saccharide antigen from serogroup A, C, W135 or Y, conjugated to a carrier protein.

6. The composition of claim 5, wherein the saccharide antigen is an oligosaccharide.

7. The composition of any preceding claim, comprising *N.meningitidis* antigens from at least two of serogroups A, C, W135 and Y.

8. The composition of any one of claims 1 to 7, wherein the carrier protein is a diphtheria or tetanus toxoid.

9. The composition of claim 8, wherein the carrier protein is CRM197.

10. The composition of any preceding claim, wherein each of the *H.influenzae* antigen, the *N.meningitidis* antigen and the *S.pneumoniae* antigen is an oligosaccharide fragment of the capsular polysaccharide, conjugated to a carrier protein.

11. The composition of claim 10, wherein the *H.influenzae* antigen is conjugated to a first carrier protein, the *N.meningitidis* antigen is conjugated to a second carrier protein and the *S.pneumoniae* antigen is conjugated to a third carrier protein.

12. The composition of claim 10, wherein the *H.influenzae* antigen, the *N.meningitidis* antigen and the *S.pneumoniae* antigen are conjugated to the same carrier protein.

13. The composition of claim 11, wherein the first, second and third carrier proteins are each separately CRM197.

14. The composition of claim 1, wherein the mucosal adjuvant is a detoxified mutant of a bacterial ADP-ribosylating toxin.

15. The composition of claim 14, wherein the mucosal adjuvant is LT-K63 or LT-R72.

16. The composition of any one of claims 1 to 15 for use in a method of raising an immune response in a patient, comprising administering to a patient the composition.

17. The composition of any one of claims 1 to 15, for use as a medicament.

18. A composition for mucosal delivery comprising a mucosal adjuvant and two or more of the following: (a) an antigen which induces an immune response against *Haemophilus influenzae,* wherein the antigen is a *Haemophilus influenzae* capsular saccharide conjugated to a carrier protein; (b) an antigen which induces an immune response against *Neisseria meningitidis,* wherein the antigen is a *Neisseria meningitidis* capsular saccharide conjugated to a carrier protein; and (c) an antigen which induces an immune response against *Streptococcus pneumoniae,* wherein the antigen is a *Streptococcus pneumoniae* capsular saccharide conjugated to a carrier protein; and further wherein the composition is for use in the induction of a protective immune response, and the mucosal adjuvant is a bacterial ADP-ribosylating toxin or a detoxified mutant thereof.

19. A process for producing the composition of any one of claims 1 to 18, comprising the steps of: (i) mixing a mucosal adjuvant and two or more of the following: (a) an antigen which induces an immune response against *Haemophilus influenzae,* wherein the antigen is a *Haemophilus influenzae* capsular saccharide conjugated to a carrier protein, (b) an antigen which induces an immune response against *Neisseria meningitidis,* wherein the antigen is a *Neisseria meningitidis* capsular saccharide conjugated to a carrier protein, and (c) an antigen which induces an immune response against *Streptococcus pneumoniae,* wherein the antigen is a *Streptococcus pneumoniae* capsular saccharide conjugated to a carrier protein; and further wherein the mucosal adjuvant is a bacterial ADP-ribosylating toxin or a detoxified mutant thereof; and (ii) formulating the mixture for mucosal delivery.

## Patentansprüche

1. Zusammensetzung zur Zuführung über die Schleimhaut, umfassend ein Schleimhaut-Adjuvans sowie mindestens zwei der folgenden Komponenten: (a) ein Antigen, das eine Immunantwort gegen *Haemophilus* influenzae induziert, wobei es sich bei dem Antigen um ein an ein Trägerprotein konjugiertes Kapselsaccharid aus *Haemophilus influenzae* handelt; (b) ein Antigen, das eine Immunantwort gegen *Neisseria meningitidis* induziert, wobei es sich bei dem Antigen um ein an ein Trägerprotein konjugiertes Kapselsaccharid aus *Neisseria meningitidis* handelt; und (c) ein Antigen, das eine Immunantwort gegen *Streptococcus pneumoniae* induziert, wobei es sich bei dem Antigen um ein an ein Trägerprotein konjugiertes Kapselsaccharid aus *Streptococcus pneumoniae* handelt; und wobei es sich ferner bei dem Schleimhaut-Adjuvans um ein bakterielles ADP-Ribosylierungstoxin oder eine detoxifizierte Mutante davon handelt.

2. Zusammensetzung nach Anspruch 1, angepasst an die intranasale Verabreichung.

3. Zusammensetzung nach Anspruch 2, in Form eines Nasensprays, von Nasentropfen, eines Gels oder eines Pulvers.

4. Zusammensetzung nach einem vorhergehenden Anspruch, wobei es sich bei dem *H.influenzae-*Kapselsaccharid-Antigen um ein Oligosaccharid handelt.

5. Zusammensetzung nach einem vorhergehenden Anspruch, wobei es sich bei dem *N.meningitidis-*Antigen um ein Kapselsaccharid-Antigen aus Serogruppe A, C, W135 oder Y, konjugiert an ein Trägerprotein, handelt.

6. Zusammensetzung nach Anspruch 5, wobei es sich bei dem Saccharid-Antigen um ein Oligosaccharid handelt.

7. Zusammensetzung nach einem vorhergehenden Anspruch, umfassend *N.meningitidis*-Antigene aus wenigstens zwei der Serogruppen A, C, W135 und Y.

8. Zusammensetzung nach einem der Ansprüche 1 bis 7, wobei es sich bei dem Trägerprotein um ein Diphtherie- oder Tetanustoxoid handelt.

9. Zusammensetzung nach Anspruch 8, wobei es sich bei dem Trägerprotein um CRM197 handelt.

10. Zusammensetzung nach einem vorhergehenden Anspruch, wobei es sich bei dem *H.influenzae-*Antigen, dem *N.meningitidis*-Antigen und dem S.pneumoniae-Antigen jeweils um ein Oligosaccharidfragment des Kapselpolysaccharids, konjugiert an ein Trägerprotein, handelt.

11. Zusammensetzung nach Anspruch 10, wobei das H.influenzae-Antigen an ein erstes Trägerprotein, das N.meningitidis-Antigen an ein zweites Trägerprotein und das S.pneumoniae-Antigen an ein drittes Trägerprotein konjugiert ist.

12. Zusammensetzung nach Anspruch 10, wobei das H.influenzae-Antigen, das N.meningitidis-Antigen und das *S.pneumoniae-*Antigen an das gleiche Trägerprotein konjugiert sind.

13. Zusammensetzung nach Anspruch 11, wobei es sich bei dem ersten, zweiten und dritten Trägerprotein jeweils unabhängig um CRM197 handelt.

14. Zusammensetzung nach Anspruch 1, wobei es sich bei dem Schleimhaut-Adjuvans um eine detoxifizierte Mutante eines bakteriellen ADP-Ribosylierungstoxins handelt.

15. Zusammensetzung nach Anspruch 14, wobei es sich bei dem Schleimhaut-Adjuvans um LT-K63 oder LT-R72 handelt.

16. Zusammensetzung nach einem der Ansprüche 1 bis 15 zur Verwendung in einem Verfahren zur Erzeugung einer Immunantwort bei einem Patienten, wobei man einem Patienten die Zusammensetzung verabreicht.

17. Zusammensetzung nach einem der Ansprüche 1 bis 15, zur Verwendung als Arzneimittel.

18. Zusammensetzung zur Zuführung über die Schleimhaut, umfassend ein Schleimhaut-Adjuvans sowie mindestens zwei der folgenden Komponenten: (a) ein Antigen, das eine Immunantwort gegen *Haemophilus influenzae* induziert, wobei es sich bei dem Antigen um ein an ein Trägerprotein konjugiertes Kapselsaccharid aus *Haemophilus influenzae* handelt; (b) ein Antigen, das eine Immunantwort gegen *Neisseria meningitidis* induziert, wobei es sich bei dem Antigen um ein an ein Trägerprotein konjugiertes Kapselsaccharid aus *Neisseria meningitidis* handelt; und (c) ein Antigen, das eine Immunantwort gegen *Streptococcus pneumoniae* induziert, wobei es sich bei dem Antigen um ein an ein Trägerprotein konjugiertes Kapselsaccharid aus *Streptococcus pneumoniae* handelt; und wobei ferner die Zusammensetzung der Verwendung bei der Induktion einer schützenden Immunantwort dient und es sich bei dem Schleimhaut-Adjuvans um ein bakterielles ADP-Ribosylierungstoxin oder eine detoxifizierte Mutante davon handelt.

19. Verfahren zur Herstellung der Zusammensetzung nach einem der Ansprüche 1 bis 18, umfassend die folgenden Schritte: (i) Mischen eines Schleimhaut-Adjuvans und mindestens zwei der folgenden Komponenten: (a) eines Antigens, das eine Immunantwort gegen *Haemophilus influenzae* induziert, wobei es sich bei dem Antigen um ein an ein Trägerprotein konjugiertes Kapselsaccharid aus *Haemophilus influenzae* handelt; (b) eines Antigens, das eine Immunantwort gegen *Neisseria meningitidis* induziert, wobei es sich bei dem Antigen um ein an ein Trägerprotein konjugiertes Kapselsaccharid aus *Neisseria meningitidis* handelt; und (c) eines Antigens, das eine Immunantwort gegen *Streptococcus pneumoniae* induziert, wobei es sich bei dem Antigen um ein an ein Trägerprotein konjugiertes Kapselsaccharid aus *Streptococcus pneumoniae* handelt; und wobei es sich ferner bei dem Schleimhaut-Adjuvans um ein bakterielles ADP-Ribosylierungstoxin oder eine detoxifizierte Mutante davon handelt; und (ii) Formulieren der Mischung zur Zuführung über die Schleimhaut.

## Revendications

1. Composition destinée à l'administration par voie muqueuse comprenant un adjuvant muqueux et deux de ce qui suit ou plus : (a) un antigène qui induit une réponse immunitaire contre *Haemophilus* influenzae, l'antigène étant un saccharide capsulaire d'*Haemophilus influenzae* conjugué à une protéine porteuse ; (b) un antigène qui induit une réponse immunitaire contre *Neisseria meningitidis,* l'antigène étant un saccharide capsulaire de *Neisseria meningitidis* conjugué à une protéine porteuse ; et (c) un antigène qui induit une réponse immunitaire contre *Streptococcus pneumoniae,* l'antigène étant un saccharide capsulaire de *Streptococcus pneumoniae* conjugué à une protéine porteuse ; et l'adjuvant muqueux étant en outre une toxine d'ADP-ribosylation bactérienne ou un mutant détoxifié de celle-ci.

2. Composition selon la revendication 1, adaptée à l'administration par voie intranasale.

3. Composition selon la revendication 2, sous forme d'un spray nasal, de gouttes nasales, de gel ou de poudre.

4. Composition selon l'une quelconque des revendications précédentes, **caractérisée en ce que** l'antigène saccharidique du saccharide capsulaire d'*H. influenzae* est un oligosaccharide.

5. Composition selon l'une quelconque des revendications précédentes, **caractérisée en ce que** l'antigène de *N. meningitidis* est un antigène de saccharide capsulaire du sérogroupe A, C, W135 ou Y, conjugué à une protéine porteuse.

6. Composition selon la revendication 5, **caractérisée en ce que** l'antigène saccharidique est un oligosaccharide.

7. Composition selon l'une quelconque des revendications précédentes, comprenant des antigènes de *N*. *meningitidis* d'au moins deux des sérogroupes A, C, W135 et Y.

8. Composition selon l'une quelconque des revendications 1 à 7, **caractérisée en ce que** la protéine porteuse est une anatoxine diphtérique ou tétanique.

9. Composition selon la revendication 8, **caractérisée en ce que** la protéine porteuse est CRM197.

10. Composition selon l'une quelconque des revendications précédentes, **caractérisée en ce que** chacun parmi l'antigène d'*H*. *influenzae,* l'antigène de *N. meningitidis* et l'antigène de *S*. *pneumoniae* est un fragment d'oligosaccharide du polysaccharide capsulaire, conjugué à une protéine porteuse.

11. Composition selon la revendication 10, **caractérisée en ce que** l'antigène d'H. *influenzae* est conjugué à une première protéine porteuse, l'antigène de *N. meningitidis* est conjugué à une deuxième protéine porteuse et l'antigène de S. *pneumoniae* est conjugué à une troisième protéine porteuse.

12. Composition selon la revendication 10, **caractérisée en ce que** l'antigène d'H. *influenzae,* l'antigène de *N*. *meningitidis* et l'antigène de *S*. *pneumoniae* sont conjugués à la même protéine porteuse.

13. Composition selon la revendication 11, **caractérisée en ce que** les première, deuxième et troisième protéines porteuses sont chacune séparément CRM197.

14. Composition selon la revendication 1, **caractérisée en ce que** l'adjuvant muqueux est un mutant détoxifié d'une toxine d'ADP-ribosylation bactérienne.

15. Composition selon la revendication 14, **caractérisée en ce que** l'adjuvant muqueux est LT-K63 ou LT-R72.

16. Composition selon l'une quelconque des revendications 1 à 15, destinée à être utilisée dans une méthode pour augmenter une réponse immunitaire chez un patient, comprenant l'administration de la composition à un patient.

17. Composition selon l'une quelconque des revendications 1 à 15, destinée à être utilisée comme médicament.

18. Composition destinée à l'administration par voie muqueuse comprenant un adjuvant muqueux et deux de ce qui suit ou plus : (a) un antigène qui induit une réponse immunitaire contre *Haemophilus* influenzae, l'antigène étant un saccharide capsulaire d'*Haemophilus influenzae* conjugué à une protéine porteuse ; (b) un antigène qui induit une réponse immunitaire contre *Neisseria meningitidis,* l'antigène étant un saccharide capsulaire de *Neisseria meningitidis* conjugué à une protéine porteuse ; et (c) un antigène qui induit une réponse immunitaire contre *Streptococcus pneumoniae,* l'antigène étant un saccharide capsulaire de *Streptococcus pneumoniae* conjugué à une protéine porteuse ; et l'adjuvant muqueux étant en outre la composition destinée à être utilisée dans l'induction d'une réponse immunitaire protectrice, et l'adjuvant muqueux étant une toxine d'ADP-ribosylation bactérienne ou un mutant détoxifié de celle-ci.

19. Procédé de préparation de la composition selon l'une quelconque des revendications 1 à 18, comprenant les étapes consistant à : (i) mélanger un adjuvant muqueux et deux de ce qui suit ou plus : (a) un antigène qui induit une réponse immunitaire contre *Haemophilus* influenzae, l'antigène étant un saccharide capsulaire d'*Haemophilus influenzae* conjugué à une protéine porteuse ; (b) un antigène qui induit une réponse immunitaire contre *Neisseria meningitidis,* l'antigène étant un saccharide capsulaire de *Neisseria meningitidis* conjugué à une protéine porteuse ; et (c) un antigène qui induit une réponse immunitaire contre *Streptococcus pneumoniae,* l'antigène étant un saccharide capsulaire de *Streptococcus pneumoniae* conjugué à une protéine porteuse ; et l'adjuvant muqueux étant en outre une toxine d'ADP-ribosylation bactérienne ou un mutant détoxifié de celle-ci ; et (ii) formuler le mélange pour l'administration par voie muqueuse.
